Europäisches Patentamt

European Patent Office

Office européen des brevets

(11) Veröffentlichungsnummer : **0 074 119**
**B1**

(12) **EUROPÄISCHE PATENTSCHRIFT**

(45) Veröffentlichungstag der Patentschrift :
20.08.86

(21) Anmeldenummer : 82108268.2

(22) Anmeldetag : 08.09.82

(51) Int. Cl.⁴ : **A 61 K 39/29, C 12 N 7/08,
G 01 N 33/576**

(54) An menschliche Fibroblastenzellen adaptierte Hepatitis-A-Viren.

(30) Priorität : 09.09.81 DE 3135599

(43) Veröffentlichungstag der Anmeldung :
16.03.83 Patentblatt 83/11

(45) Bekanntmachung des Hinweises auf die Patenterteilung : 20.08.86 Patentblatt 86/34

(84) Benannte Vertragsstaaten :
AT BE CH DE FR GB IT LI LU NL SE

(56) Entgegenhaltungen :
EP-A- 0 025 745
DE-A- 3 033 406
DE-B- 1 037 655
DE-C- 1 037 656
FR-A- 2 398 504
GB-A-   839 876
GB-A-   878 704
GB-A- 1 105 179
GB-A- 1 498 821
NL-A- 6 611 493
US-A- 2 915 436
US-A- 3 000 788
US-A- 3 432 391
US-A- 4 164 566
BIOLOGICAL ABSTRACTS, Band 73, 1982, Nr. 36393
V. GAUSS-MUELLER et al.: "Propagation of hepatitis
A virus in human embryo fibroblasts. Human diploid
fibroblasts and human primary liver carcinoma cells"
BIOLOGICAL ABSTRACTS, Band 74, 1982, Nr. 7254 B.
FLEHMIG et al.: "Hepatitis A virus in cell culture: 3.
Propagation of hepatits A virus in human embryo
kidney cells and human embryo fibroblasts strains"
Die Akte enthält technische Angaben, die nach dem
Eingang der Anmeldung eingereicht wurden und die
nicht in dieser Patentschrift enthalten sind

(73) Patentinhaber : Flehmig, Bertram, Dr.
Horemer 21
D-7400 Tübingen (DE)

(72) Erfinder : Flehmig, Bertram, Dr.
Horemer 21
D-7400 Tübingen (DE)

(74) Vertreter : Patentanwälte Zellentin
Zweibrückenstrasse 15
D-8000 München 2 (DE)

**Beschreibung**

In der deutschen Patentanmeldung P 30 33 406.6 ist ein Verfahren zur Herstellung von Hepatitis-A-Viren (HAV) beschrieben. Dort gelingt es erstmals, das Wachstum der HAV dadurch zu beschleunigen, daß man HAV auf Rhesusaffennierenzellen (FrhR-4/R) aussät, die nach mehreren Wochen erscheinenden Viren erntet und erneut aussät.

Dieser Vorgang wird solange wiederholt, bis die Viren eine befriedigende Wachstumsgeschwindigkeit aufweisen. Die dort gewonnenen HAV sind zur Herstellung von Vaccinen für die Humanmedizin ungeeignet, da sie von Tierzellen stammen.

Aus Journal of Medical Virologie, 7 (3) 233-239, April 1981 sind an menschliche Fibroblastenzellen adaptierte HAV bekannt, die durch direkte Anzucht von klinischen oder zellkulturadaptierten HAV auf Fibroblastenzellen unter Selektion von innerzellulären Viren gewonnen wurden. Bei der Adaption werden 90 bis 210 und mehr Tage benötigt, bis die ersten Viren nachweisbar sind.

Aus EP-A1-25 745 ist ferner ein Verfahren bekannt, bei dem HAV vor der Passage in diploiden Fibroblastenzellen durch 5 bis 25 Passagen in Nierenzellen von Affen an derartige Zellen adaptiert wurden. Zur Selektion werden innerzelluläre Viren verwendet, die durch Einfrieren, Auftauen und Beschallen aus den Zellen gewonnen werden.

Derartige an menschliche Fibroblastenzellen (MFZ) adaptierte HAV weisen aufgrund ihrer Selektion über innerzelluläre Viren den Nachteil auf, daß sie stets unter Zerstörung der Zellen geerntet werden müssen, da die Anzahl der in die Nährlösung ausgeschleusten Viren nicht hoch genug ist.

Die Viren gemäß EP-A1-25 745 weisen ferner den Nachteil auf, daß ihre Verwendung für die Humanmedizin wegen des intensiven vorausgehenden Kontakts mit Affennierenzellen nicht möglich oder sehr bedenklich ist.

Die vorliegende Erfindung hat sich die Aufgabe gestellt, HAV herzustellen, die bedenkenlos in der Humanmedizin Verwendung finden können und außerdem auf vorteilhafte Weise adaptiert und geerntet werden können.

Diese Aufgabe wird durch an menschliche Fibroblastenzellen (MFZ) adaptierte Hepatitis-A-Viren (HAV), erhalten durch Züchtung von aus Stuhl isoliertem HAV auf menschlichen Embryonierenzellen (MENZ), Infizieren von MFZ mit dabei in das Nährmedium ausgeschleusten Viren und Isolierung der in das MFZ-Nährmedium ausgeschleusten Viren, sowie durch ein Verfahren zur ihrer Gewinnung sowie ihre Verwendung gelöst.

Derartige Viren sind erfindungsgemäß über menschliche Embryonierenzellen (MENZ) an menschliche Fibroblastenzellen (MFZ) adaptierte Hepatitis-A-Viren : HAV/MENZ/MFZ, die beim Paul-Ehrlich-Institut, Frankfurt/Main unter dem Namen Dr. Bertram Flehmig hinterlegt sind (wissenschaftliche Bezeichnung : HAV/GBM/HFS).

Ihre Herstellung gelingt auf folgendem Wege : Aus Stuhl isoliertes HAV wird in MENZ gezüchtet. Man bedient sich dabei bekannter Methoden, wie sie z. B. auch in der Patentanmeldung P 30 33 406 beschrieben sind. Die ersten, nach etwa 7 bis 8 Wochen in das Nährmedium ausgeschleusten Viren werden verwendet, um erneut MENZ zu infizieren (Passage 2 in MENZ). Man wiederholt diesen Vorgang über etwa 10 Passagen, wodurch sich die Wachstumsgeschwindigkeit jeweils steigert. Die schnell und in großen Mengen wachsenden Viren werden dabei jeweils selektiert.

Man erhält so erstmals an MENZ adaptierte HAV (HAV/MENZ).

Es muß dabei als ausgesprochen überraschend angesehen werden, daß

1. nach einem vergleichsweise sehr großen Zeitraum überhaupt Viren geerntet werden können. Die übliche Wachstumsgeschwindigkeit liegt erheblich höher, so daß frühzeitig von einer Unwahrscheinlichkeit eines derartigen Vorgangs gesprochen werden kann,

2. eine Adaption mit hoher Wachstumsgeschwindigkeit an MENZ durch mehrfaches Passieren erreicht werden kann.

Diese Verhältnisse wiederholen sich bei der Infizierung der menschlichen Fibroblastenzellen (MFZ) mit HAV/MENZ. Auch hier wächst die erste Passage des HAV in MFZ ausgesprochen langsam, so daß auch hier ein Erfolg überraschen muß.

Durch mehrfaches Passieren von HAV/MENZ auf MFZ werden so letztlich an MFZ adaptierte HAV, HAV/MENZ/MFZ, erhalten, die sowohl aus der Zelle als auch aus dem Nährmedium geerntet werden können.

Diese sind in gleicher Weise zur Verwendung in Tests mit HAV Antikörpern, vor allem aber zur Herstellung von Impfstoffen für die Humanmedizin geeignet.

In den nachfolgenden Beispielen wird die Erfindung näher erläutert :

1. Herstellung von menschlichen Embryonierenzellen (MENZ) und menschlichen Embryofibroblastenzellen (MFZ).

Die Herstellung von MENZ erfolgt aus den Nieren von menschlichen Feten, die Herstellung von MFZ aus den Lungen menschlicher Feten. Die Aufarbeitung der Organe erfolgt nach allgemein bekannten Bedingungen. Subpassagen der ersten Monolayerschicht von MENZ sind 5- bis 10mal möglich, bei MFZ ca. 50- bis 75mal. Teile der ersten Passage von MENZ als auch von MFZ können nach bekannten Methoden in — 70 ºC

oder flüssigen Stickstoff eingefroren werden und bei Bedarf wieder aufgetaut und verwendet werden.

2. Züchtung von HAV in MENZ und MFZ.

Dicht gewachsene Monolayerschichten von MENZ und MFZ werden mit HAV-haltigen Suspensionen inkubiert, die Viren werden dadurch an die Zellen adsorbiert. Danach wird ein geeignetes Nährmedium auf die Zellen gegeben und die Zellen werden bei 34 bis 37 °C inkubiert. Nach entsprechenden Inkubationszeiten wird die Menge an HAV, die in das Nährmedium der Zellen aus diesen ausgeschleust wurde, durch geeignete Nachweisverfahren bestimmt. Das ausgeschleuste HAV kann nun für Testverfahren zum Nachweis von Antikörpern in menschlichen oder tierischen Seren verwendet werden, oder aber nach bestimmten Reinigungs- und Inaktivierungsverfahren als Vaccine bei Mensch und Tier.

**Patentansprüche**

1. An menschliche Fibroblastenzellen (MFZ) adaptierte Hepatitis-A-Viren (HAV), erhalten durch Züchtung von aus Stuhl isoliertem HAV auf menschlichen Embryonierenzellen (MENZ), Infizieren von MFZ mit dabei in das Nährmedium ausgeschleusten Viren und Isolierung der in das MFZ-Nährmedium ausgeschleusten Viren.

2. Verfahren zur Adaption von HAV an MFZ, dadurch gekennzeichnet, daß man aus Stuhl isoliertes HAV auf MENZ züchtet und mit in das Nährmedium ausgeschleusten Viren MFZ infiziert.

3. Verfahren nach Anspruch 2, dadurch gekennzeichnet, daß man die Wachstumsgeschwindigkeit der HAV auf MENZ dadurch steigert, daß man die zunächst erst nach etwa 7 bis 8 Wochen im Nährmedium erscheinenden ersten HAV erntet und erneut auf MENZ aussät und diesen Vorgang mehrfach wiederholt, wodurch die Wachstumsdauer je Passage etwa um 1 bis 2 Wochen gesenkt wird, und daß die so gewonnenen, an MENZ adaptierten HAV in gleicher Weise durch mehrfaches Passieren an MFZ adaptiert werden.

4. Verwendung von über MENZ an MFZ adaptierten HAV nach Anspruch 1 bzw. den HAV-Antigenen zur Reaktion mit Antikörpern in Testsystemen oder zur Herstellung von Vaccinen zur Immunisierung von Mensch oder Tier.

**Claims**

1. To human fibroblast cells (HFC) adapted hepatitis-A-viruses (HAV), obtained by the cultivation on human embryo kindney cells (HEKC) of HAV isolated from stools, infecting of HFC with viruses transferred into the nutrient medium and isolating the viruses transferred into the HFC nutrient medium.

2. Method for adapting HAV to HFC, characterised in that HAV, isolated from stools, is cultivated on HEKC and is infected with viruses which are transferred into the nutrient medium.

3. Method according to claim 2 characterised in that the growth rate of HAV on HEKC is increased by first harvesting the first HAV, which appear after approximately 7 to 8 weeks in the nutrient medium, and are again seeded on HEKC and this process is repeated several times, whereby the growth time per pass is reduced by approximately 1 to 2 weeks and that the HAV thus obtained and adapted to HEKC is in like manner adapted to HFC by repeated passes.

4. Application of HAV, adapted through HEKC to HFC according to claim 1 or to the antigens for reaction with antibodies in the test system or for the production of vaccines for humans or animals.

**Revendications**

1. Virus de l'hépatite A (VHA) adaptés à des cellules fibroblastiques humaines (CFH), obtenus par culture de VHA, isolés à partir de selles, sur des cellules embryorénales humaines (CERH), infection de CFH avec des virus passés dans le milieu de culture, et isolation des virus passés dans le milieu de culture des CFH.

2. Procédé d'adaptation de VHA à des CFH, caractérisé en ce qu'on cultive des VHA sur des CERH et qu'on infecte des CFH avec des virus passés dans le milieu de culture.

3. Procédé selon la revendication 2, caractérisé en ce qu'on augmente la vitesse de multiplication des VHA sur des CERH en recueillant d'abord les premiers VHA se présentant après environ 7-8 semaines dans le milieu de culture, et en les semant ensuite de nouveau sur des CERH, ce processus étant répété à plusieurs reprises, la durée de multiplication étant ainsi raccourcie d'environ 1 à 2 semaines à chaque stade de passage, et en ce que les VHA ainsi recueillis et adaptés aux CERH sont, de manière analogue, adaptés aux CFH également par des passages multiples.

4. Application des VHA, adaptés par l'entremise des CERH à des CFH selon la revendication 1, ou les antigènes de VHA, à la réaction avec des anticorps dans des systèmes d'essai, ou bien à la préparation de vaccins destinés à l'immunisation de l'homme ou de l'animal.